# EUROPÄISCHE PATENTSCHRIFT

(11) **EP 1 318 154 B1**
(45) Veröffentlichungstag und Bekanntmachung des Hinweises auf die Patenterteilung: **16.06.2004**
(21) Anmeldenummer: 01129137.4
(22) Anmeldetag: 07.12.2001
(51) Int. Cl.: C07F 9/10, C12Q 1/44

(54) **Verbindungen zur Bestimmung der Aktivität von Phospholipase A2**
Compounds for determination of the activity of phospholipase A2
Composés pour la détermination de l'activité de la phospholipase A2

(43) Veröffentlichungstag der Anmeldung: 11.06.2003
(73) Patentinhaber: SIRS-Lab GmbH, 07745 Jena (DE)
(72) Erfinder: Deigner, Hans-Peter, Dr., 69514 Laudenbach (DE); Russwurm, Stefan, Dr., 07743 Jena (DE); Kinscherf, Ralf, Dr., 69198 Schriesheim (DE); Lösche, Wolfgang, Dr., 99096 Erfurt (DE)
(74) Vertreter: Störle, Christian, Dr.

(56) Entgegenhaltungen:
- HENDRICKSON H STEWART ET AL: "Intramolecularly quenched BODIPY-labeled phospholipid analogs in phospholipase A2 and platelet-activating factor acetylhydrolase assays and in vivo fluorescence imaging" ANALYTICAL BIOCHEMISTRY, ACADEMIC PRESS, SAN DIEGO, CA, US, Bd. 276, Nr. 1, 1. Dezember 1999 (1999-12-01), Seiten 27-35, XP002186412 ISSN: 0003-2697
- CHEMICAL ABSTRACTS, vol. 118, no. 19, 10. Mai 1993 (1993-05-10) Columbus, Ohio, US; abstract no. 192186, KUSUNOKI, CHIHIRO ET AL: "Preparation of phosphatidylethanolamine derivatives as substrates for phospholipase A2 determination" XP002200175 & JP 04 282391 A (FUJISAWA PHARMACEUTICAL CO., LTD., JAPAN) 7. Oktober 1992 (1992-10-07)
- BAYBURT T. ET AL.: "Continuous, vesicle-based fluorimetric assays of 14- and 85-kDa phospholipases" ANALYTICAL BIOCHEMISTRY., Bd. 232, Nr. 1, - 20. November 1995 (1995-11-20) Seiten 7-23, XP002200174 ACADEMIC PRESS INC. NEW YORK., US ISSN: 0003-2697
- THUREN T. ET AL.: "Phospholipase A2 assay using an intramolecularly quenched pyrene- labelled phospholipid analog as a substrate" ANALYTICAL BIOCHEMISTRY., Bd. 170, Nr. 1, - April 1988 (1988-04) Seiten 248-255, XP008003510 ACADEMIC PRESS INC. NEW YORK., US ISSN: 0003-2697

## Beschreibung

Die Erfindung bezieht sich auf Verbindungen, die zur Bestimmung der Aktivität von Phospholipase A₂ (PLA₂), insbesondere der Platelet-Activating Factor Acetylhydrolase (PAF-AH), geeignet sind.

Der Platelet-Activating Factor (PAF) ist ein biologisch aktives Phospholipid, der an physiologischen und pathophysiologischen Prozessen beteiligt ist. Dieser Faktor wird durch eine spezifische Acetylhydrolase, dem Platelet-Activating Factor Acetylhydrolase (PAF-AH, EC 3.1.1.47, auch als Lipoprotein associated phospholipase A₂ bezeichnet), welche die Acetatgruppe in sn-2 Position abspaltet, in biologisch inaktive Produkte umgewandelt. Die Stubstratspezifität umfaßt neben PAF auch oxidierte Phospholipide mit polaren Substituenten in ω-Position des *sn*-2 Esters. Diese veränderten Substanzen werden häufig in oxidierten Lipoproteinen angetroffen. Da neben PAF und den oxidierten Phospholipiden auch die oxidierten Lipoproteine eine Rolle bei den unterschiedlichsten Krankheiten aufweisen, ist davon auszugehen, daß auch das Enzym einen entscheidenden Einfluß auf die Genese dieser Krankheiten hat.

In den letzten Jahren ist PAF-AH immer stärker in den Vordergrund der Forschung getreten. Insbesondere wurden Abnormalitäten der PAF-AH-Aktivität bei unterschiedlichsten Krankheiten festgestellt. Klinische Testreihen und der Beginn der Untersuchungen des rekombinant hergestellten Enzyms als Therapeutikum (Die Phase III der klinischen Untersuchungen beginnt im Frühjahr 2001 auf dem Indikationsgebiet Sepsis (www.icos.com)) zeigen die große Bedeutung für den Menschen. Es ist deshalb erforderlich, ein gut durchführbares Verfahren zur Aktivitätsbestimmung von PLA₂ allgemein und PAF-AH im besonderen zu Verfügung zu haben.

Zur Bestimmung der Aktivität von PAF-AH werden in der Literatur die unterschiedlichsten Verfahren beschrieben, wie Radioaktivitätsmessungen, UV - oder Fluoreszenz - Bestimmungen. Bei nahezu allen dieser Verfahren schließen sich nach der eigentlichen Umsetzung des Substrates mit einer Probe, in der das Enzym enthalten ist, ein oder mehrere Aufarbeitungsschritt(e) (z.B. Extraktion, Folgereaktionen zu Indikatoren) an, eine kontinuierliche Messung der Enzymkinetik ist nicht möglich.

Die ältesten und zur Zeit am meisten eingesetzten Bestimmungsmethoden basieren auf Radioaktivitätsmessungen (M.E. Blank et al., J. Biol. Chem, 1981, 256, 175-178). Dabei wird ein mit [³H]-acetat markiertes natürliches Substrat mit der zu untersuchenden Probe umgesetzt, wobei radioaktiv markierte Essigsäure abgespalten wird. Nach chromatographischer Aufarbeitung des Testansatzes kann die abgespaltene Essigsäure radioaktiv bestimmt werden. Eine chromatographische Aufarbeitung ist aber zeitaufwendig und auch mit zum Teil hohen Kosten verbunden.

Aus H.S. Hendrickson et al., Anal. Biochem., (1999), 276, 27-35 sind intramolekular gequenchte BODIPY-markierte Phospholipidanaloga in Verfahren zur Bestimmung von PLA₂ und PAF-AH bekannt. Eines dieser Analoga ist 1-(*N*-(BODIPY-FL-pentanoyl)-11-aminoundecyl)-2-((*N*-(2,4-dinitrophenyl)amino)-8-amino-octanoyl)-*sn*-glycero-3-phosphocholin. BODIPY fungiert als Fluorophohr.

Diese Verbindung weist aber ganz gravierende Nachteile auf. So ist bei Verwendung vorgenannter Verbindung zur Bestimmung der Aktivität von PLA₂, insbesondere PAF-AH, eine Aussage über Veränderungen der Lateraldiffusionsgeschwindigkeit in Membranen nicht möglich. Außerdem weichen die Eigenschaften vorgenannter Verbindung stark von den Eigenschaften natürlicher Phospholipide ab.

Der vorliegenden Erfindung liegt somit die Aufgabe zugrunde, ein Mittel bereitzustellen, mit dem die Aktivität von PLA₂, insbesondere PAF-AH, ohne die aus dem Stand der Technik bekannten Nachteile bestimmt werden kann.

Erfindungsgemäß wird dies erreicht durch eine Verbindung der Formel (1) 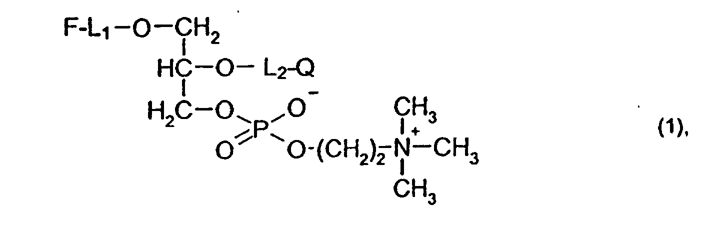 in der
L₁ von einem Ether (R¹-OR²)ₘ, wobei R¹ und R² unabhängig voneinander gewählt sind und von einem Kohlenwasserstoff mit 1 bis 12 Kohlenstoffatomen stammen, wobei m eine ganze Zahl von 1 bis 4 ist, oder von einem Kohlenwasserstoff R mit 1 bis 20 Kohlenstoffatomen abgeleitet ist;
F für ein unsubstituiertes oder substituiertes Pyren als Fluorophor;
Q für einen Quencher, und
L₂ für C(O)-L₁ oder C(O)-L₁-NH, wobei L₁ wie vorstehend definiert ist, stehen.

Obwohl die erfindungsgemäßen Verbindungen ein unsubstituiertes oder substituiertes Pyren als Fluorophor aufweisen, fluoreszieren sie nicht, da die Fluoreszenz durch den ebenfalls in der erfindungsgemäßen Verbindung enthaltenen Quencher gelöscht wird. Bei Einwirken von PLA₂, insbesondere PAF-AH, wird der Quencher abgespalten und von der übrig bleibenden Verbindung räumlich entfernt. Dadurch wird die Quenchung der Fluoreszenz aufgehoben und dadurch eine Zunahme der Intensität der Fluoreszenz beobachtet. Diese Zunahme der Intensität der Fluoreszenz ist im allgemeinen direkt proportional zur Aktivität von PLA₂ und somit ein Indikator für die Aktivität von PLA₂, insbesondere PAF-AH.

Es wurde nun überraschenderweise gefunden, daß bei Verwendung der erfindungsgemäßen Verbindungen zur Bestimmung der Aktivität von PLA₂, insbesondere PAF-AH, Aussagen über Veränderungen der Lateraldiffusionsgeschwindigkeit gemacht werden können. Außerdem weichen die Eigenschaften der erfindungsgemäßen Verbindungen wenn überhaupt dann nur unwesentlich von den Eigenschaften von natürlichen Phospholipiden ab. Des weiteren ist mit den erfindungsgemäßen Verbindungen eine Aktivitätsbestimmung in besonders effektiver und einfach durchzuführender Weise möglich. Die Aktivität kann direkt und kontinuierlich gemessen werden.

Wie vorstehend ausgeführt wurde, liegt in der erfindungsgemäßen Verbindung ein unsubstituiertes oder substituiertes Pyren als Fluorophor vor. Dieses kann über seine 1-Position an L₁ gebunden sein. Ist das Pyren substituiert, werden solche Substituenten eingesetzt, die die Eigenschaften als Fluorophor nicht wesentlich beeinflussen. Solche Substituenten sind dem Fachmann bekannt. Sie können Amino- oder Carbonyl-Gruppen oder Heteroatome sein, wie N, S oder P, die C-Atome von Pyren substituieren. Die erfindungsgemäße Verbindung kann einen oder mehrere, unabhängig voneinander gewählte Substituenten aufweisen.

Gegenüber anderen Fluorophoren, wie NBD (*N*-(7-Nitrobenz-2-oxa-1,3-diazol-4-yl)) oder BODIPY (4,4-Difluoro-4-bora-3a,4a-diaza-s-indacen) haben vorstehende Pyrene die Vorteile einer geringeren Polarität, eines großen Abstandes der Extinktions- und Emissionswellenlänge, der Möglichkeit in wäßrigen Lösungen zu arbeiten, eine günstige Verteilung in Zellen, die Fähigkeit zur Einlagerung in Membranen ohne Quenchung der Fluoreszenz sowie eine pHunabhängige Fluoreszenz. Eine weitere positive Eigenschaft von Pyrenen ist die Bildung von Eximeren bei höheren Konzentrationen und einer gleichzeitigen Verschiebung der Emissionswellenlänge zu höheren Wellenlängen. Durch die Eximerbildung ist eine Beurteilung der Konzentration des Fluorophors möglich.

Die erfindungsgemäßen Verbindungen weisen gemäß vorstehender Formel (1) einen Quencher auf. Dabei handelt es sich um im Bereich der Fluoreszenz an sich bekannte Verbindungen, die die Fluoreszenzintensität des Fluorophors reduzieren bzw. löschen. Beispiel solcher Quencher sind Phenylreste, die mit einer oder mehreren, insbesondere zwei, Nitrogruppen substituiert sind, wie beispielsweise der 2,4-Dinitrophenyl-Rest. Mit diesen Quenchem ist eine intramolekulare Reduktion bzw. Quenchung der Fluoreszenzintensität der Fluorophore in besonders effektiver Weise möglich.

In den erfindungsgemäßen Verbindungen ist das Fluorophor über einen Linker an das O-Atom in sn 1-Position des Glycerins gebunden. Der Quencher ist ebenfalls über einen Linker an das Glycerin gebunden, und zwar an *sn*-2 Position des Glycerins. Die Linker werden dabei hinsichtlich ihrer Dimensionen, insbesondere der Länge, so gewählt, daß eine optimale Quenchung der Fluoreszenzintensität zu beobachten ist. Der Linker L₂, durch den der Quencher an den Glycerin-Rest gebunden ist, ist wegen der Gruppierung O-C(O) durch PLA₂, insbesondere PAF-AH, hydrolytisch spaltbar. Durch die Hydrolyse wird der Quencher zusammen mit dem Linker aus der erfindungsgemäßen Verbindung abgespalten; die Fluoreszenzintensität nimmt dann zu, da eine intramolekulare Quenchung nicht mehr möglich ist. Der Linker L₁, durch den das Fluorophor an das Glycerin gebunden wird, darf dagegen durch PLA₂, insbesondere PAF-AH, nicht gespalten werden. Vielmehr muß zum Nachweis der Fluoreszenzintensität der Linker metabolisch stabil sein.

Wie bereits vorstehend ausgeführt wurde, kann der Linker von einem Ether (R¹-OR²)ₘ abgeleitet werden, wobei R¹ und R² unabhängig voneinander gewählt sind und von einem Kohlenwasserstoff mit 1 bis 12 Kohlenstoffatomen stammen, wobei m eine ganz Zahl von 1 bis 4 ist.

Mit dem Begriff "Kohlenwasserstoff" werden organische Verbindungen bezeichnet, die nur aus Kohlenstoff und Wasserstoff bestehen. Beispiele dafür sind Alkane, Alkene und Alkine. Bei Alkenen und Alkinen können mehrere ungesättigte Bindungen vorliegen. Gemäß vorstehender Formel (1) sind von vorgenannten Kohlenwasserstoffen zwei H-Atome substituiert, damit sie ihre Funktion als bzw. im Linker erfüllen können. Diese Substitution kann insbesondere an terminalen C-Atomen der Kohlenwasserstoffen erfolgen.

In einer bevorzugten Ausführungsform der erfindungsgemäßen Verbindung stehen R¹ und R² für (CH₂)ₙ, wobei n eine ganze Zahl von 2 bis 12 ist. Insbesondere steht L₁ für (CH₂)₄-O-(CH₂)₁₀. Linker mit CH₂-Ketten sind besonders günstig, da sie metabolisch besonders stabil sind, wodurch gewährleistet wird, daß trotz enzymatischer Behandlung das Fluorophor in der erfindungsgemäßen Verbindung verbleibt und zur Fluoreszenz angeregt werden kann.

Wie vorstehend ausgeführt wurde, kann der Linker L₁ von einem Kohlenwasserstoff R abgeleitet sein. Vorzugsweise steht R für (CH₂)ₒ, wobei o eine ganze Zahl von 1 bis 20, insbesondere 2 bis 6, ist. Linker dieser Art haben sich als besonders günstig erwiesen, da sie metabolisch sehr stabil sind, wodurch eine unerwünschte Abspaltung des Fluorophors aus der erfindungsgemäßen Verbindung bei Behandlung mit Enzymen vermieden wird.

Die erfindungsgemäße Verbindung weist einen zweiten Linker L₂ auf, mit dem der Quencher an die *sn*-2 Position des Glycerins gebunden ist. Wie vorstehend ausgeführt wurde, steht L₂ für C(O)-L₁ oder C(O)-L₁-NH, wobei L₁ wie vorstehend definiert ist.

In einer bevorzugten Ausführungsform steht L₂ für C(O)-(CH₂)ₚ oder C(O)-(CH₂)ₚ-NH, wobei p eine ganze Zahl von 1 bis 20, insbesondere 2 bis 6 ist, insbesondere bedeutet L₂ C(O)-(CH₂)₅-NH. Diese Linker sind besonders gut geeignet, da auf der einen Seite die CH₂-Kette metabolisch stabil ist, wodurch eine unerwünschte Abspaltung des Quenchers an dieser Stelle vermieden wird, auf der anderen Seite aber die Ester-Gruppe -C(O)-O- von PLA₂, insbesondere PAF-AH, gezielt und selektiv durch diese Enzyme gespalten wird.

Ein Vorteil, Pyren an Position 1 des Glycerins zu binden, ist, daß es dort metabolisch stabil ist; es sind keine Störungen des Tests durch Phospholipase A1 Aktivitäten, die möglicherweise auch in der zu untersuchenden Probe vorliegen, festzustellen.

An *sn* 3-Position des Glycerins befindet sich der Phosphatidylcholin-Rest. Dadurch wird in günstiger Weise sichergestellt, daß die erfindungsgemäßen Verbindungen das biophysikalische Verhalten natürlicher Phospholipide zeigen.

In einer besonders bevorzugten Ausführungsform weist die erfindungsgemäße Verbindung die Struktur der Formel (2) auf

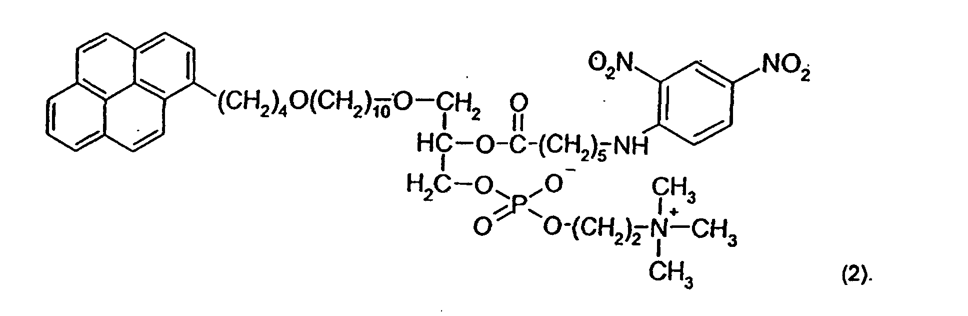

Mit der erfindungsgemäßen Verbindung gemäß der Formel (2) ist es in besonders einfacher und günstiger Weise möglich, die Aktivität von PLA₂, insbesondere PAF-AH, zu messen.

Zur Herstellung der erfindungsgemäßen Verbindung kann der Fachmann auf übliche Reaktionen und Verfahren in der synthetischen organischen Chemie zurückgreifen. Beispielsweise kann zunächst das Fluorophor mit einem Linker verbunden werden, anschließend kann das zweite Endes des Linkers in die sn-1 Position des Glycerins eingeführt werden. Dann kann in die sn-3 Position des Glycerins der Phosphocholin-Rest eingebracht werden, wobei die OH-Gruppe an der sn-2 Position des Glycerins durch eine Schutzgruppe, beispielsweise eine Benzoyl-Gruppe, geschützt werden kann. Die Schutzgruppe kann nachfolgend in üblicher Weise abgespalten werden und dann der zweite Linker, gegebenenfalls zusammen mit dem Quencher, eingefügt werden. Das Verfahren zur Herstellung der erfindungsgemäßen Verbindungen wurde vorstehend ohne die detaillierte Beschreibung von Zwischenschritten beschrieben. Beispielsweise kann das Einbringen des Linkers in das Fluorophor über mehrere Schritte erfolgen, wie sie beispielsweise im nachfolgenden Herstellungsbeispiel beschrieben sind.

Durch die Anwesenheit des Quenchers in der erfindungsgemäßen Verbindung erfolgt eine intramolekulare Quenchung der Fluoreszenz. Durch Abspaltung des Quenchers von der erfindungsgemäßen Verbindung ist keine intramolekulare Quenchung mehr möglich, wodurch eine Zunahme der Fluoreszenzintensität des Fluorophors zu beobachten ist. Eine solche Abspaltung des Quenchers von der erfindungsgemäßen Verbindung kann durch PLA₂; insbesondere PAF-AH, erfolgen, und zwar durch Hdyrolyse der Ester-Gruppe an sn-2 Position. Die erfindungsgemäßen Verbindungen sind deshalb besonders gut zur Bestimmung der Aktivität von PLA₂, insbesondere PAF-AH, geeignet.

Bei einem solchen Verfahren zur Bestimmung der Aktivität von PLA₂, insbesondere PAF-AH, wird eine dieses Enzym enthaltende Probe mit der erfindungsgemäßen Verbindung inkubiert und in üblicher Weise die Fluoreszenz gemessen. Die Inkubation erfolgt dabei günstigerweise unter physiologischen Bedingungen, beispielsweise pH 7,4 in einem wäßrigen Medium, z.B. Puffer, und 37°C, um die Aktivität des Enzyms in möglichst natürlicher Umgebung messen zu können. Die Messung der Fluoreszenz, d.h. die Zunahme der Fluoreszenz, kann kontinuierlich erfolgen. Bei einer solchen kontinuierlichen Messung kann die Fluoreszenz andauemd über einen längeren Zeitraum von wenigen Minuten bis einigen Stunden, insbesondere 5 Minuten bis 0,5 Stunden bestimmt werden.

Die erfindungsgemäßen Verbindungen sind besonders gut zur Bestimmung der Enzyme PLA₂, insbesondere PAF-AH, geeignet. Durch diese Verbindungen ist eine besonders effektive, direkte, einfache, schnelle und kostengünstige Messung möglich. Des weiteren kann die Aktivitätsmessung kontinuierlich, d.h. über einen längeren Zeitraum andauernd, durchgeführt werden, wodurch wertvolle Informationen zu Enzymkinetik erhalten werden können. Des weiteren werden mit den erfindungsgemäßen Verbindungen sehr genaue Werte erhalten, wodurch die gerade für therapeutische und diagnostische Zwecke zuverlässigen Aussagen getroffen werden können.

Die Aktivitätsbestimmung der Enzyme kann eine ganze Anzahl von Anwendungen haben, beispielsweise
- als Diagnostikum und zur Differentialdiagnose aus unterschiedlichsten biologischen Proben bei Mensch und Tier (Nasensekret, Sperma, Lungensekret (BAL), Blut bzw. dessen Bestandteilen, isolierten Zellpopulationen, Gewebebiopsien, Gallensekret, Darmsekret, Stuhlproben wie
   - zum Nachweis verschiedenster Stoffwechselfunktionen z.B. unspezifischen Zuständen wie Hyperlipdämie, Zuständen von erhöhtem oxidativen Streß;
   - zur Diagnose von akuten Entzündungserkrankungen, wie Sepsis, ARDS (Lungenversagen), sowie Entzündungen solitärer Organe sowie insbesondere auch von Wundheilungsstörungen;
   - zum Nachweis von chronischen Entzündungszuständen wie Rheumatoide Arthritis und weitere Erkrankungen des rheumatischen Formenkreises, Colitis ulzerosa, Morb. Crohn;
   - bei sonstigen lokalen und systemischen Entzündungszuständen;
   - bei allergischen und/oder immonologisch vermittelten Erkrankungen, wie z.B. Pollenund Latexallergie, Glomerulonephritis;
   - bei Erkrankungen toxischer Genese, wie z.B. toxisches Lungenversagen oder Vergiftungszustände;
   - bei koronaren Erkrankungen wie beispielsweise akutes Koronarsyndrom, Angina pectoris, Prinzmetall-Angina, Herzinfarkt sowie Arteriosklerose, akutem cerebralen Insult;

   - bei sonstigen Zuständen wie nach Transplantationen, Operationen, sonst. Invasiven Prozeduren;
   - im Rahmen der Vorsorgeuntersuchung gegen o.g. medizinische Krankheitsbilder;
- zum Therapiemonitoring ärztlicher Massnahmen, wie
   - jeglicher medikamentöser Interventionen wie von Therapien mit rekombinanten Enzymen sowie aller therapeutischer Interventionen welche mit Aktivitätsänderungen der entspr. Enzyme einhergehen; insbesondere in den o.g. therapeutischen Indikationsfeldem;
   - invasiver Massnahmen wie Operationen zur Beseitigung entzündlicher Ursachen, Operationen mit Ersatz oder mechanischer Unterstützung der Herz-Lungen-Funktion, Operationen an der Leber und auch verschiedenster Transplantationen; insbesondere in den o.g. therapeutischen Indikationsfeldern;
   - der Kombination von beidem.

Die Bestimmung der Aktivität von PLA₂, insbesondere PAF-AH mit erfindungsgemäßen Verbindungen weist eine ganze Reihe von Vorteilen auf. So kann die Aktivitätsbestimmung des Enzyms ohne lästige Aufarbeitungsschritte und ohne Nebenreaktionen erfolgen. Durch die Zugabe von geeigneten Inhibitoren ist eine selektive Messung von Phospholipid-Esterspaltenden Enzymen in Gemischen verschiedener Hydrolasen möglich. Die Enzymaktivitätsbestimmung mit den erfindungsgemäßen Verbindungen kann in den Vertiefungen von Mikrotiterplatten erfolgen. Ferner ist diese Aktivitätsbestimmung HTS-fähig. Durch die kontinuierliche Messung ist es möglich, kinetische Daten, z.B. IC50- und KI-Werte, zu erfassen.

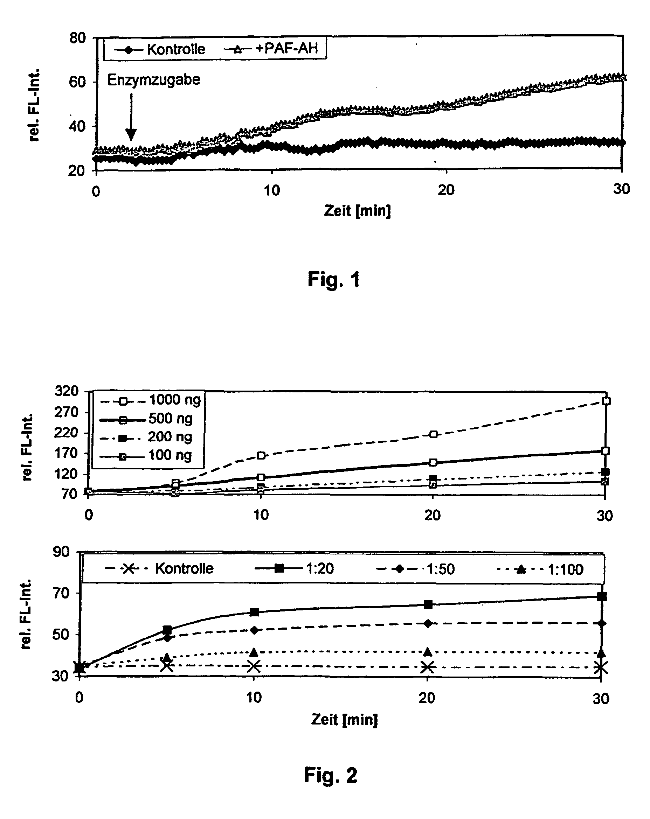

Die folgenden Beispiele erläutern unter Bezugnahme auf die Figuren 1 und 2 die vorliegende Erfindung, ohne sie aber darauf einzuschränken, wobei
- Fig. 1: den typischen zeitlichen Verlauf der Fluoreszenzzunahme durch die Einwirkung von PAF-AH und
- Fig. 2: den Zusammenhang zwischen unterschiedlichen Mengen an Enzym- bzw. unterschiedlichen Verdünnungen von humanem Probenmaterial unter Zunahme der Fluoreszenz zeigen.

### Beispiel 1: Herstellung der erfindungsgemäßen Verbindung 1-O-{10-[4-(Pyren-1-yl)-butoxy]-decyl}-2-(6-(2,4-dinitrophenyl)-aminohexoyl)-rac-glycero-3-phosphocholin

### Dichlorophosphorsäure-(2-bromoethylester)

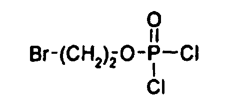

Zu einer Lösung von 42.5 g (0.275 mol) Phosphorylchlorid in 50 ml Dichlormethan wurden 27 g (0.215 mol) Bromethanol in 15 ml Dichlormethan innerhalb von 15 min bei Raumtemperatur getropft. Nach 15 min wurde für 18 Stunden ein leichter Argonstrom durch die Reaktionslösung geleitet. Durch Destillation im Ölpumpenvakuum (73 - 74 °C, 3 * 10⁻² mbar) wurden 50.5 g (76%) einer farblosen Flüssigkeit erhalten.
M (C₂H₄⁷⁸Br³⁵Cl₂O₂³¹P) = 239 g/mol
EI-MS: m/z (%) = 243 (0.5) [M], 241 (0.9) [M], 239 (0.6) [M], 163 (25), 161 (41), 149 (18), 147 (30), 137 (36), 135 (55), 119 (23), 117 (34), 109 (64), 108 (100), 107 (69), 106 (100), 99 (18), 47 (27). ¹H-NMR (CDCl₃): δ (ppm) = 4.59 (2H, m, C*H*₂Br, ³J_{HH} = 6.26 Hz, ³J_{HP} = 10.34 Hz), 3.13 (2H, m, C*H*₂O, ³J_{HH} = 6.26 Hz, ⁴J_{HP} = 1.05 Hz). ¹³C-NMR (CDCl₃): δ (ppm) = 69.88 (1 CH₂O, ²J_{CP} = 8.39 Hz), 27.32 (1 *C*H₂Br, ³J_{CP} = 10.29 Hz). IR (Film): 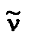 (cm⁻¹) = 3033*w*, 2971*w*, 2883*w*, 1453*m*, 1423*m*, 1385*w*, 1306*s*, 1233*m*, 1180*m*, 1071*s*, 1016*s*, 963*m*, 908*m*, 845*w*, 759*m*.

### 4-Oxo-4-(pyren-1-yl)-buttersäure

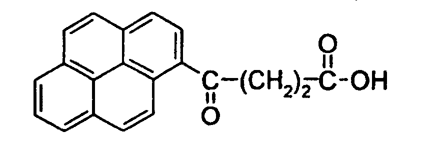

Zu einer Lösung von 0.5 g (5 mmol) Bernsteinsäureanhydrid in 60 ml Nitrobenzol wurden bei 0°C 1.32 g (5 mmol) wasserfreies AlCl₃ und 1 g (5 mmol) Pyren hinzugefügt. Nach einer Reaktionszeit von 18 Stunden bei Raumtemperatur wurde die Lösung auf 30 ml eiskalter Salzsäure (25 %ig) gegossen, wobei sich ein Niederschlag bildete. Nach Abfiltration und Umkristallisation aus EtOH wurden 1.233 g (82 %) 4-Oxo-4-(pyren-1-yl)-buttersäure erhalten.
M (C₂₀H₁₄O₃) = 302 g/mol
EI-MS: m/z (%) = 303 (10) [MH⁺], 302 (42) [M], 230 (16), 229 (100), 202 (11), 201 (65), 200 (24). HR-EI-MS (C₂₀H₁₄O₃): ber. 302.0943; gef. 302.0943. ¹H-NMR ([D₆]-DMSO): δ (ppm) = 8.81 - 8.12 (9H, m, C*H*ₐᵣₒₘ), 3.52 (2H, t, COC*H*₂, ³J_{HH} = 6.22 Hz), 2.82 (2H, t, C*H*₂CO₂H, ³J_{HH} = 6.22 Hz). ¹³C-NMR ([D₆]-DMSO): δ (ppm) = 203.05 (1 *C*O), 173.87 (1 *C*O₂H), 132.97 - 123.41 (9 *C*Hₐᵣₒₘ, 7 *C*ₐᵣₒₘ), 36.87 (1 CO*C*H₂), 28.52 (1 *C*H₂CO₂H). IR (KBr): 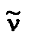 (cm⁻¹) = 3426*m*, 3039*m*, 2984*m*, 2929*m*, 2677*w*, 2594*w*, 1695*s*, 1665*s*, 1507*w*, 1404*w*, 1212*m*, 1123*w*, 1075*w*, 842*m*, 713*w*.

### 4-(Pyren-1-yl)-buttersäure

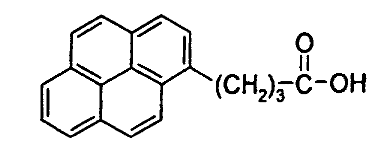

1 g (2.78 mmol) 4-Oxo-4-(pyren-1-yl)-buttersäure wurden in 30 ml Diethylenglycol gelöst und mit 0.5 g (10 mmol) Hydrazinhydrat sowie 0.56 g (10 mmol) KOH versetzt. Die Reaktionslösung wurde 2 Stunden unter Rückfluß erhitzt und sodann auf eiskalte Salzsäure (25 %ig) gegossen, wobei sich ein gelber Niederschlag bildete. Nach Abfiltrieren des Feststoffes und Umkristallisation aus EtOH wurden 570 mg (71 %) 4-(Pyren-1-yl)-buttersäure erhalten.
M (C₂₀H₁₆O₂) = 288 g/mol
EI-MS: m/z (%) = 289 (16) [MH⁺], 288 (62) [M], 216 (20), 215 (100), 213 (11). HR-EI-MS (C₂₀H₁₆O₂): ber. 288.1150; gef. 288.1150. ¹H-NMR ([D₆]-DMSO): δ (ppm) = 8.50 - 7.92 (9H, m, C*H*ₐᵣₒₘ), 3.36 (2H, m, C*H*₂C₁₆H₉), 2.41 (2H, m, C*H*₂CO₂H), 2.04 (2H, m, C*H*₂). ¹H-NMR (CDCl₃): δ (ppm) = 8.32 - 7.86 (9H, m, C*H*ₐᵣₒₘ), 3.42 (2H, t, C*H*₂C₁₆H₉, ³J_{HH} = 7.57 Hz), 2.51 (2H, t, C*H*₂CO₂H, ³J_{HH} = 7.00 Hz), 2.23 (2H, m, C*H*₂, ³J_{HH} = 7.57 Hz, ³J_{HH} = 7.00 Hz). ¹³C-NMR ([D₆]-DMSO): δ (ppm) = 174.74 (1 *C*O), 136.39 - 123.39 (9 *C*Hₐᵣₒₘ, 7 *C*ₐᵣₒₘ), 33.64 - 26.98 (1 *C*H₂CO₂H, 2 *C*H₂). ¹³C-NMR (CDCl₃): δ (ppm) = 177.48 (1 *C*O), 135.25 - 123.04 (9 *C*Hₐᵣₒₘ, 7 *C*ₐᵣₒₘ), 33.21 - 26.46 (1 *C*H₂CO₂H, 2 *C*H₂). IR (KBr): 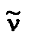 (cm⁻¹) = 3447*w*, 3037*w*, 2950*m*, 2934*w*, 2874*w*, 1695*s*, 1431*w*, 1275*m*, 1206*m*, 918*w*, 846*s*, 711*w*.

### 4-(Pyren-1-yl)-buttersäuremethylester

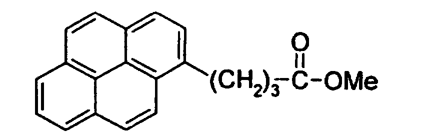

40 ml Methanol wurde bei 0°C tropfenweise mit 20 ml (260 mmol) Thionylchlorid versetzt. Anschließend wurde die Lösung 1 h bei Raumtemperatur gerührt. Nach Hinzufügen von 5.50 g (19 mmol) 4-(Pyren-1-yl)-buttersäure wurde der Ansatz Ober Nacht bei Raumtemperatur gerührt. Die Reaktion wurde mit gesättigter NaHCO₃-Lösung neutralisiert und mit Essigsäureethylester extrahiert. Aus der organischen Phase wurde ein Aliquot zur Analytik entnommen. Das restliche Rohprodukt wurde zur Darstellung des 4-(Pyren-1-yl)-butan-1-ol weiterverwendet.
M (C₂₁H₁₈O₂) = 302 g/mol
EI-MS: m/z (%) = 303 (13) [MH⁺], 302 (57) [M], 228 (15), 216 (22), 215 (100), 213 (12), 108 (13). HR-EI-MS (C₂₁H₁₈O₂): ber. 302.1307; gef. 302.1306. ¹H-NMR (CDCl₃): δ (ppm) = 8.25 - 7.70 (9H, m, C*H*ₐᵣₒₘ), 3.63 (3H, s, C*H*₃), 3.27 (2H, t, C*H*₂C₁₆H₉, ³J_{HH} = 7.60 Hz), 2.36 (2H, t, C*H*₂CO, ³J_{HH} = 7.20 Hz), 2.11 (2H, m, C*H*₂, ³J_{HH} = 7.20 Hz, ³J_{HH} = 7.60 Hz). ¹³C-NMR (CDCl₃): δ (ppm) = 173.73 (1 CO), 135.51 - 123.15 (9 *C*Hₐᵣₒₘ, 7 *C*ₐᵣₒₘ), 51.38 (1 *C*H₃), 33.52 - 26.64 (1 *C*H₂CO, 2 *C*H₂). IR (KBr): 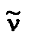 (cm⁻¹) = 3033*w*, 2949*w*, 2876*w*, 1734*s*, 1429*m*, 1209*m*, 1161*m*, 842*s*.

### 4-(Pyren-1-yl)-butan-1-ol

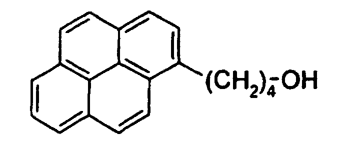

Das Rohprodukt der Vorstufe wurde bei 0°C in 30 ml THF gelöst und mit 3 g (79 mmol) LiAlH₄ versetzt. Die Reaktion wurde über Nacht bei Raumtemperatur gerührt. Anschließend wurde das überschüssige Reduktionsmittel hydrolisiert. Nach säulenchromatischer Aufarbeitung wurden 4.6 g (92 %, über 2 Stufen) der gewünschten Verbindung erhalten.
M (C₂₀H₁₈O) = 274 g/mol
EI-MS: m/z (%) = 275 (10) [MH⁺], 274 (53) [M], 216 (17) 215 (100). HR-EI-MS (C₂₀H₁₈O): ber. 274.1358; gef. 274.1357. ¹H-NMR (CDCl₃): δ (ppm) = 8.23 - 7.79 (9H, m, C*H*ₐᵣₒₘ), 3.64 (2H, t, C*H*₂O, ³J_{HH} = 6.39 Hz), 3.31 (2H, t, C*H*₂C₁₆H₉, ³J_{HH} = 7.60 Hz), 1.87 (2H, m, C*H*₂, ³J_{HH} = 7.70 Hz, ³J_{HH} = 7.60 Hz), 1.69 (2H, m, C*H*₂, ³J_{HH} = 7.70 Hz, ³J_{HH} = 6.39 Hz). ¹³C-NMR (CDCl₃): δ (ppm) = 136.65 -123.35 (9 C*H*ₐᵣₒₘ, 7 *C*ₐᵣₒₘ), 62.77 (1 *C*H₂O), 33.17 - 27.93 (3 *C*H₂). IR (KBr): 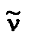 (cm⁻¹) = 3423*s*,*br*, 3038*m*, 2927*m*, 2859*m*, 1181*w*, 1030*w*, 982*w*, 841*s*.

### 1-[4-(10-Bromo-decyloxy)-butyl]-pyren

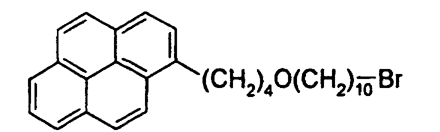

2 g (7.3 mmol) 4-(Pyren-1-yl)-butan-1-ol wurden in 50 ml Toluol mit 1 g (9 mmol) Kalium-*tert*-butylat bei 100°C 1 Stunden erhitzt. Anschließend wurden 2.22 g (7.4 mmol) 1,10-Dibromdecan zugefügt und weitere 16 Stunden erhitzt. Nach säulen-chromatographischer Aufarbeitung wurden 2.82 g (79 %) eines hell gelben Feststoffes erhalten.
M (C₃₀H₃₇O⁷⁸Br) = 492 g/mol
EI-MS: m/z (%) = 495 (10) [MH⁺], 494 (31) [M], 493 (10) [MH⁺], 492 (33) [M], 412 (13), 228 (22), 215 (100), 55 (18), 41 (13). HR-EI-MS (C₃₀H₃₇O⁷⁸Br): ber. 492.2028; gef. 492.2028. ¹H-NMR (CDCl₃): δ (ppm) = 8.30 - 7.85 (9H, m, C*H*ₐᵣₒₘ), 3.49 - 3.45 (2H, t, C*H*₂Br, ³J_{HH} = 6.44 Hz), 3.41 - 3.34 (6H, m, C*H*₂C₁₆H₉, 2 C*H*₂O, ³J_{HH} = 7.08 Hz, ³J_{HH} = 6.81 Hz), 1.96 - 1.90 (2H, m, C*H*₂, ³J_{HH} = 7.08 Hz, ³J_{HH} = 6.44 Hz), 1.83 - 1.73 (4H, m, 2 C*H*₂, ³J_{HH} = 6.81 Hz), 1.55 - 1.53 (2H, m, C*H*₂), 1.25 (12H, m, 6 C*H*₂). ¹³C-NMR (CDCl₃): δ (ppm) = 136.72 123.30 (9 C*H*ₐᵣₒₘ, 7 *C*ₐᵣₒₘ), 70.93 - 70.55 (2 *C*H₂O), 33.94 - 26.15 (1 *C*H₂Br, 11 *C*H₂). IR (KBr): 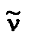 (cm⁻¹) = 3037*w*, 2924*s*, 2851*s*, 1499*m*, 1448*m*, 1429*w*, 1369*w*, 1243*w*, 1184*w*, 1117*s*, 1063*w*, 965*w*, 849*m*, 841*s*, 710*s*.

### 2,2-Dimethyl-4-[10-O-(4-(pyren-1-yl)-butoxy)-decyl)-methyl]-[1,3]-dioxolan

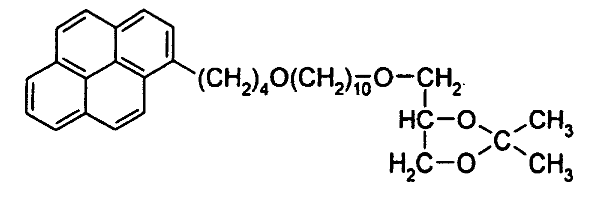

1.85 g (14 mmol) 2,2-Dimethyl-4-hydroxymethyl-1,3-dioxolan wurden in 80 ml Toluol mit 1.6 g (14 mmol) Kalium-*tert*-butylat 1 Stunden auf Rückfluß erhitzt. Nach Abkühlen auf Raumtemperatur wurden 2.118 g (4.3 mmol) 1-[4-(10-Bromo-decyloxy)-butyl]-pyren hinzugefügt und für weitere 18 Stunden erhitzt. Nach säulenchromatographischer Aufarbeitung wurden 1.174 g (50 %) eines hell gelben Festoffes erhalten.
M (C₃₆H₄₈O₄) = 544 g/mol
EI-MS: mlz (%) = 545 (22) [MH⁺], 544 (62) [M], 257 (22), 228 (24), 216 (22), 215 (100), 101 (18), 55 (11), 43 (14). HR-EI-MS (C₃₆H₄₈O₄): ber. 544.3553; gef. 544.3553. ¹H-NMR (CDCl₃): δ (ppm) = 8.29 - 7.83 (9H, m, C*H*ₐᵣₒₘ), 4.26 - 4.21 (2H, m, CHC*H*₂O), 4.07 - 4.00 (1H, m, C*H*O), 3.75 - 3.31 (10H, m, 4 OC*H*₂, C*H*₂C₁₆H₉), 1.95 - 1.86 (2H, m, C*H*₂), 1.78 - 1.72 (2H, m, C*H*₂), 1.55 - 1.53 (4H, m, C*H*₂), 1.41 und 1.35 (6H, s, 2 C*H*₃), 1.26 (12H, m, 6 C*H*₂). ¹³C-NMR (CDCl₃): δ (ppm) = 136.92 -123.48 (9 *C*Hₐᵣₒₘ, 7 *C*ₐᵣₒₘ), 109.33 (1 *C*(CH₃)₂), 74.77 (1 CHO), 71.85 - 70.64 (4 *C*H₂O), 66.94 (1 *C*H₂O), 33.33 - 25.89 (11 *C*H₂), 26.77 und 25.43 (2 *C*H₃). IR (KBr): 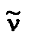 (cm⁻¹) = 3059*m*, 2952*s*, 2890*m*, 2866*s*, 1524*w*, 1467*m*, 1369*w*, 1289*m*, 11121*m*, 1058*m*, 837*w*, 684*s*.

### 1-O-{10-[4-(Pyren-1-yl)-butoxy]-decyl}-rac-glycerin

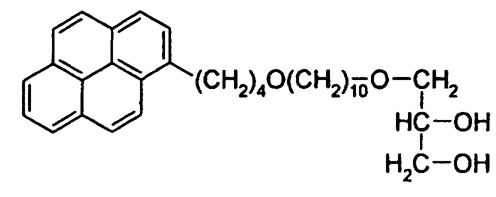

0.183 g (0.336 mmol) 2,2-Dimethyl-4-[10-*O*-(4-(pyren-1-yl)-butoxy)-decyl)-methyl]-[1,3]-dioxolan wurden in 10 ml Toluol bei 0°C mit 1 ml Trifluoressigsäure versetzt und eine Stunde gerührt. Nach Neutralisation mit wässriger NaHCO₃-Lösung wurden nach säulenchromatographischer Aufarbeitung 0.160 g (94 %) eines hell gelben Festoffes erhalten.
M (C₃₃H₄₄O₄) = 504 g/mol
EI-MS: m/z (%) = 505 (32) [MH⁺], 504 (100) [M], 257 (17), 228 (21), 216 (19), 215 (89), 55 (14). HR-EI-MS (C₃₃H₄₄O₄): ber. 504.3240; gef. 504.3238. ¹H-NMR (CDCl₃): δ (ppm) = 8.29 - 7.83 (9H, m, C*H*ₐᵣₒₘ), 3.87 - 3.59 (3H, m, C*H*OH, C*H*₂OH), 3.47 - 3.31 (10H, m, 4 OC*H*₂, C*H*₂C₁₆H₉), 1.94 1.89 (2H, m, C*H*₂), 1.77 -1.72 (2H, m, C*H*₂), 1.54 - 1.51 (4H, m, 2 C*H*₂), 1.24 (12H, m, 6 C*H*₂). ¹³C-NMR (CDCl₃): δ (ppm) = 136.94 - 123.48 (9 *C*Hₐᵣₒₘ, 7 *C*ₐᵣₒₘ), 72.03 - 70.65 (4 *C*H₂O), 70.50 (1 *C*HOH), 63.81 (1 *C*H₂OH), 33.31 - 25.97 (11 *C*H₂). IR (KBr): 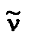 (cm⁻¹) = 3419s,*br*, 3058*w*, 2932*m*, 2851*m*, 1437*m*, 1433*m*, 1265*m*, 1205*m*, 1186*m*, 1138*m*, 1112*m*, 1048*m*, *843m.*

### 1-O-{10-[4-(Pyren-1-yl)-butoxy]-decyl}-3-O-triphenylmethyl-rac-glycerin

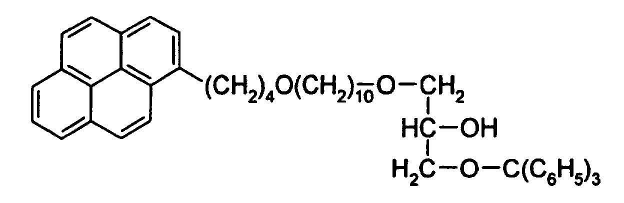

4.215 g (8.4 mmol) 1-*O*-{10-[4-(Pyren-1-yl)-butoxy]-decyl}-*rac*-glycerin wurden in 50 ml Acetonitril vorgelegt und 4.09 g (10 mmol) 1-(Triphenylmethyl)pyridinium-tetrafluoroborat in 20 ml Acetonitril versehen. Die Lösung wurde 18 Stunden gerührt, eingeengt und in Chloroform aufgenommen. Der Feststoff wurde abfiltriert. Aus dem Filtrat wurden nach Säulenchromatographie 3.697 g (59 %) der gewünschte Verbindung als hellgelber Feststoff erhalten.
M (C₅₂H₅₈O₄) = 746 g/mol
FAB-MS (FAB⁺, NBA): m/z = 746.4 [M], 504.3, 244.1, 215.1. HR-FAB (C₅₂H₅₈O₄): ber.: 746.4335; gef.: 746.4375. HR-FAB (C₅₂H₅₈O₄Na): ber.: 769.4233; gef.: 769.4304. ¹H-NMR (CDCl₃): δ (ppm) = 8.31 - 7.85 (9H, m, C*H*ₐᵣₒₘ), 7.45 - 7.19 (15H, m, C*H*ₐᵣₒₘ), 3.95 (1H, m, C*H*), 3.55 - 3.33 (10H, m, 4 OC*H*₂, C*H*₂C₁₆H₉), 3.24 - 3.18 (2H, m, C*H*₂OC(C₆H₅)₃), 1.97 - 1.87 (2H, m, C*H*₂), 1.81 - 1.73 (2H, m, C*H*₂), 1.55 - 1.51 (4H, m, 2 C*H*₂), 1.25 (12H, m, 6 C*H*₂). ¹³C-NMR (CDCl₃): **δ** (ppm) = 146.90 123.51 (24 *C*Hₐᵣₒₘ, 10 *C*ₐᵣₒₘ), 86.65 (1 *C*(C₆H₅)₃), 72.95 - 70.67 (4 *C*H₂O), 69.87 (1 *C*H), 64.65 (1 *C*H₂OC(C₆H₅)₃), 33.35 - 26.97 (11 *C*H₂). IR (KBr): 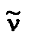 (cm⁻¹) = 3429*m*, 3056*w*, 2927*s*, 2855*s*, 1602*w*, 1490*m*, 1448*m*, 1319*w*, 1213*m*, 1113*m*, 1077*m*, 1033*m*, 899*w*, 846*m*, *746m,* 706*s*.

### 1-O-{10-[4-(Pyren-1-yl)-butoxy]-decyl}-2-O-benzoyl-3-O-triphenylmethyl-rac-glycerin

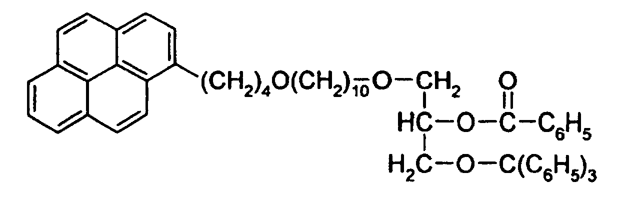

Zu einer Lösung von 2.578 g (3.455 mmol) 1-*O*-{10-[4-(Pyren-1-yl)-butoxy]-decyl}-3-*O*-triphenylmethyl-*rac*-glycerin und 1.5 ml (18.66 mmol) Pyridin in 50 ml CH₂Cl₂ wurden bei 0°C 540 mg (3.857 mmol) Benzoylchlorid hinzugetropft und 1 Stunde bei Raumtemperatur gerührt. Nach säulenchromatographischer Aufarbeitung wurden 2.8 g (95 %) des Produktes isoliert.
M (C₅₉H₆₂O₅) = 850 g/mol
FAB-MS (FAB⁺, NBA): m/z = 873.4 [M+Na], 850.4 [M], 608.3, 591.3, 391.3, 307.1, 243.1. 215.1. ¹H-NMR (CDCl₃): δ (ppm) = 8.30 - 7.86 (9H, m, C*H*ₐᵣₒₘ), 7.45 - 7.21 (20H, m, C*H*ₐᵣₒₘ), 5.46 - 5.41 (1H, m, C*H*), 3.79 - 3.34 (12H, m, 4 C*H*₂O, C*H*₂C₁₆H₉, C*H*₂OC(C₆H₅)₃), 1.97 1.89 (2H, m, C*H*₂), 1.80 - 1.72 (2H, m, C*H*₂), 1.55 -1.51 (4H, m, C*H*₂), 1.25 (12H, m, C*H*₂). ¹³C-NMR (CDCl₃): δ (ppm) = 166.00 (1 *C*O), 146.88 123.51 (29 *C*Hₐᵣₒₘ, 11 *C*ₐᵣₒₘ), 86.47 (1 *C*(C₆H₅)₃), 72.65 (1 *C*H), 71.59 - 69.47 (4 *C*H₂O), 62.86 (1 *C*H₂OC(C₆H₅)₃), 33.35 - 26.01 (11 *C*H₂). IR (KBr): 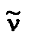 (cm⁻¹) = 3057*w*, 2927*s*, 2854*m*, 1718*s*, 1448*m*, 1384*m*, 1272*m*, 1212*w*, 1177*w*, 1158*w*, 1112*m*, 1032*w*, 899*w*, 846*m*, 763*m*, 703*m*.

### 1-O-{10-[4-(Pyren-1-yl)-butoxy]-decyl}-2-O-benzoyl-rac-glycerin

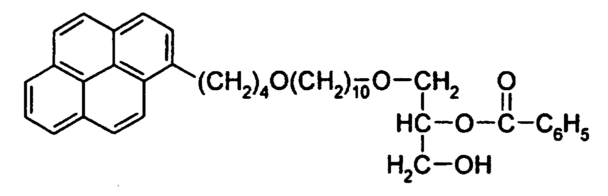

0.55 g (0.647 mmol) 1-*O*-{10-[4-(Pyren-1-yl)-butoxy]-decyl}-2-*O*-benzoly-3-*O*-tri-phenylmethyl*rac*-glycerin wurden in 20 ml CH₂Cl₂ gelöst und bei 10°C mit 0.5 ml BF₃ (13 %ig in MeOH) tropfenweise versetzt. Nach einer Stunden wurde die Reaktion mit 5 ml eiskaltem Wasser abgestoppt. Nach säulenchromatographischer Aufarbeitung wurden 280 mg (71 %) der Verbindung erhalten.
M (C₄₀H₄₈O₅) = 608 g/mol
FAB-MS (FAB⁺, NBA): m/z = 608.3 [M], 591.3, 469.3, 329.1, 257.2, 215.1. HR-FAB (C₄₀H₄₈O₅): ber.: 608.3502; gef.: 608.3519. HR-FAB (C₄₀H₄₈O₅Na): ber.: 631.3399; gef.: 631.3424. ¹H-NMR (CDCl₃): δ (ppm) = 8.29 - 7.84 (9H, m, C*H*ₐᵣₒₘ), 7.62 - 7.34 (5H, m, C*H*ₐᵣₒₘ), 5.27 - 5.22 (1H, m, C*H*), 3.99 - 3.34 (12H, m, 4 C*H*₂O, C*H*₂C₁₆H₉, C*H*₂OH), 1.97 1.88 (2H, m, C*H*₂), 1.80 - 1.72 (2H, m, C*H*₂), 1.57 - 1.51 (4H, m, 2 C*H*₂), 1.25 (12H, m, 6 C*H*₂). ¹³C-NMR (CDCl₃): δ (ppm) = 166.38 (1 *C*O), 136.94 - 123.51 (14 *C*Hₐᵣₒₘ, 8 *C*ₐᵣₒₘ), 73.81 (1 *C*H), 71.94 - 68.95 (4 *C*H₂O), 62.87 (1 *C*H₂OH), 33.80 - 26.19 (11 *C*H₂). IR (KBr): 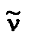 (cm⁻¹) = 3431*m*, 3038*m*, 2930*s*, 2859*m*, 1718*s*, 1603*m*, 1585*m*, 1453*m*, 1435*m*, 1317*m*, 1274*s*, 1179*m*, 1113*m*, 1027*m*, 936*m*, 846*m*, 709*s*.

### 1-O-{10-[4-(Pyren-1-yl)-butoxy]-decyl}-2-O-benzoyl-rac-glycero-3-phosphocholin

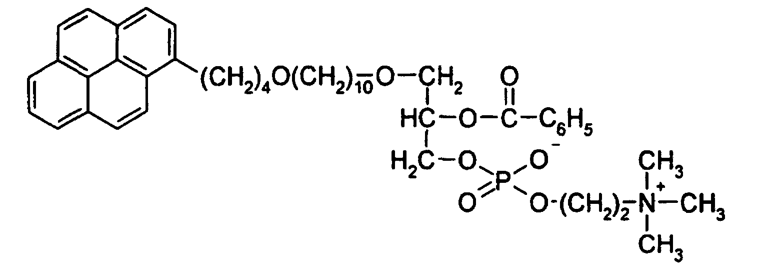

0.55 g (2.3 mmol) 2-Bromoethyl-dichlorophosphat wurden zu 15 ml CH₂Cl₂ bei 0°C gegeben und mit einer Mischung aus 1.6 ml Pyridin und 4 ml CH₂Cl₂ tropfenweise versetzt. Sodann wurden 210 mg (3.45 mmol) 1-*O*-{10-[4-(Pyren-1-yl)-butoxy]-decyl}-2-*O*-benzoyl-*rac*-glycerin in 2 ml CH₂Cl₂ gelöst und bei Raumtemperatur zu der Phosphorylierungsmischung getropft. Die Reaktion wurde für 2 Stunden auf Rückfluß erhitzt und über Nacht bei Raumtemperatur gerührt. Nach Hydrolyse mit 5 ml Eiswasser (1 Stunde Nachrühren) wurde die Reaktion mit CHCl₃ /MeOH 3:2 ausgeschüttelt. Die organische Phase wurde über Na₂SO₄ getrocknet, einrotiert und in 15 ml CHCl₃, 10 ml Acetonitril und 5 ml *iso*-Propanol gelöst. Anschließend wurden 1.5 g Trimethylamin-Lösung (33 %ig in Ethanol) hinzugegeben. Der Reaktionskolben wurde dicht verschlossen und über Nacht bei 50°C gerührt. Die Reaktionslösung wurde einrotiert, mit 5 ml CHCl₃ aufgenommen und jeweils zweimal mit 4 ml Ameisensäure / MeOH (5:7), 4 ml Natriumacetat (0.1 M) / MeOH (5:7) und 3 ml Natriumchlorid (1 M) / MeOH (5:7) ausgeschüttelt. Aus der organischen Phase wurde nach Na₂SO₄-Trocknung und Säulenchromatographie 99 mg (38 %) der gewünschten Verbindung erhalten .
M (C₄₅H₆₀N₂O₈³¹P) = 773 g/mol
FAB-MS (FAB⁺, NBA): m/z = 796.3 [M+Na], 774.4 [MH⁺], 601.5, 569.4, 531.4, 413.3, 215.1. HR-FAB (C₄₅H₆₁O₈NP): ber.: 774.4145; gef.: 774.4135. HR-FAB (C₄₅H₆₀O₈NPNa): ber.: 796.3954; gef.: 796.3951. ¹H-NMR (CD₃OD): δ (ppm) = 8.34 - 7.89 (9H, m, C*H*ₐᵣₒₘ), 7.73 - 7.45 (5H, m, C*H*ₐᵣₒₘ), 5.41 - 5.37 (1CH, m, C*H*), 4.20 - 4.17 (2H, m, C*H*₂N), 4.05 - 3.35 (14H, m, 4 OC*H*₂, C*H*₂OP, POC*H*₂, C*H*₂C₁₆H₉), 3.16 (9H, s, 3 C*H*₃), 1.96 - 1.90 (2H, m, C*H*₂), 1.81 - 1.72 (2H, m, C*H*₂), 1.52 - 1.48 (4H, m, 2 C*H*₂), 1.28 (12H, m, 6 C*H*₂). ¹³C-NMR (CD₃OD): δ (ppm) = 167.78 (1 *C*O), 138.30 - 124.60 (14 *C*Hₐᵣₒₘ, 8 *C*ₐᵣₒₘ), 74.27 (1 *C*H), 72.81 - 70.48 (4 *C*H₂O), 68.87, 66.90, 60.95 (1 *C*H₂N, 1 *C*H₂OP, 1 PO*C*H₂), 54.90 (3 *C*H₃), 34.38 - 27.29 (11 *C*H₂).

### 1-O-{10-[4-(Pyren-1-yl)-butoxy]-decyl}-rac-glycero-3-phosphocholin

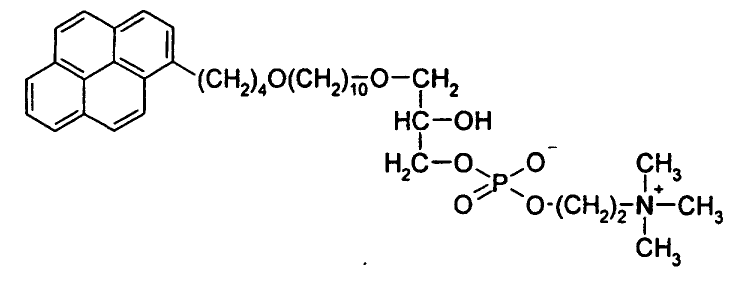

Es wurden 90 mg (0.12 mmol) 1-*O*-{10-[4-(Pyren-1-yl)-butoxy]-decyl}-2-*O*-benzoyl-*rac*-glycero-3-phosphocholin in 3 ml MeOH gelöst und mit 1.5 ml einer 0.1 M Bu₄NOH-Lösung in MeOH (1.25 eq) versehen. Die Reaktionslösung wurde nach 2 Stunden eingeengt. Nach säulenchromatographischer Aufarbeitung wurden 71 mg (90 %) der lyso-Verbindung erhalten.
M (C₃₈H₅₆NO₇³¹P) = 669 g/mol
FAB-MS (FAB⁺, NBA): m/z = 692.3 [M+Na], 670.4 [MH⁺], 669.4 [M], 584.3, 242.3, 289.1, 215.1. HR-FAB (C₃₈H₅₇NO₇³¹P): ber.: 670.3873; gef.: 670.3881. HR-FAB (C₃₈H₅₆NO₇³¹PNa): ber.: 692.3692; gef.: 692.3693. ¹H-NMR (CD₃OD): δ (ppm) = 8.23 - 7.88 (9H, m, C*H*ₐᵣₒₘ), 4.43 - 4.38 (2H, m, C*H*₂N), 4.02 - 3.36 (15H, m, 4 OC*H*₂, OC*H*, C*H*₂OP, POC*H*₂, C*H*₂C₁₆H₉), 3.22 (9H, s, 3 C*H*₃), 1.96 -1.87 (2H, m, C*H*₂), 1.77 1.66 (2H, m, C*H*₂), 1.55 -1.47 (4H, m, 2 C*H*₂), 1.27 (12H, m, 6 C*H*₂). ¹³C-NMR (CDCl₃): δ (ppm) = 170.46 (1 *C*O), 138.16 - 124.48 (9 *C*Hₐᵣₒₘ, 7 *C*ₐᵣₒₘ), 72.90 - 71.47 (4 *C*H₂O), 71.03 (1 *C*H), 67.31, 66.63, 59.33 (1 *C*H₂OP, 1 *C*H₂N, 1 PO*C*H₂), 54.78 (3 *C*H₃), 33.36 - 24.59 (10 *C*H₂). IR (KBr): 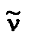 (cm⁻¹) = 3418*s*, 3039*m*, 2852*m*, 1635*m*, 1490*m*, 1467*m*, 1375*w*, 1230*m*, 1111*m*, 1087*m*, 1051*m*, 970*m*, 928*w*, 850*m*.

### 1-O-{10-[4-(Pyren-1-yl)-butoxy]-decyl}-2-(6-(2,4-dinitrophenyl)-aminohexoyl)-rac-glycero-3-phosphocholin

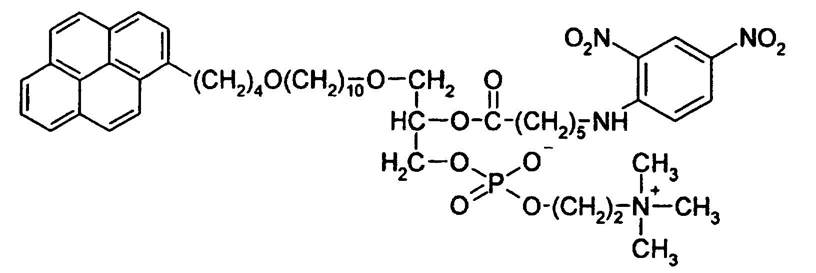

0.150 g (0.22 mmol) 1-*O*-{10-[4-(Pyren-1-yl)-butoxy]-decyl}-*rac*-glycero-3-phosphocholin wurden in 10 ml Dichlormethan mit 0.326 g (1.1 mmol) 6-(2,4-Dinitrophenylamino)-hexansäure, 136 mg (0.66 mmol) DCC und 408 mg (3.2 mmol) DMAP versehen und 40 Stunden bei Raumtemperatur gerührt. Die entstandene Suspension wurde sodann mit 5 ml Wasser versehen und über Kieselgur filtriert. Nach Trennung der Phasen und chromatographischer Aufarbeitung wurden 56 mg (26 %) des Produktes isoliert.
M (C₅₀H₆₉N₄O₁₂³¹P) = 948 g/mol
FAB-MS (FAB⁺, NBA): m/z = 971.4 [M+Na], 949.4 [MH⁺], 884.4, 862.4, 721.4, 243.2, 215.1. HR-FAB (C₅₀H₇₀O₁₂N₄P): ber.: 949.4725; gef.: 949.4755. HR-FAB (C₅₀H₆₉O₁₂N₄PNa): ber.: 971.4545; gef.: 971.4547. ¹H-NMR (CDCl₃): δ (ppm) = 9.00 - 6.67 (12H, m, C*H*ₐᵣₒₘ), 5.13 - 5.10 (1H, m, C*H*), 4.34 - 4.28 (2H, m, C*H*₂N), 4.08 - 3.23 (16H, m, 4 OC*H*₂, POC*H*₂, C*H*₂OP, C*H*₂N, C*H*₂C₁₆H₉), 3.18 (9H, s, 3 CH₃), 2.38 - 2.31 (2H, m, C*H*₂CO), 1.95 -1.86 (4H, m, 2, C*H*₂), 1.80 - 1.72 (2H, m, C*H*₂), 1.56 - 1.41 (8H, m, 4 C*H*₂), 1.25 (12H, m, 6 C*H*₂). ¹³C-NMR (CDCl₃): δ (ppm) = 170.46 (1 *C*O), 148.66 - 113.98 (12 *C*Hₐᵣₒₘ, 10 *C*ₐᵣₒₘ), 73.86 (1 *C*H), 72.81 - 69.12 (4 *C*H₂O), 67.31, 66.63, 60.30 (1 *C*H₂OP, 1 *C*H₂N, 1 PO*C*H₂), 54.78 (3 *C*H₃), 43.27 (1 *C*H₂N), 33.36 - 24.59 (1 *C*H₂CO, 14 *C*H₂). IR (KBr): 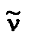 (cm⁻¹) = 3365*m*, 3107*w*, 3042*w*, 2928*m*, 2853*m*, 1729*m*, 1623*s*, 1589*m*, 1525*m*, 1500*w*, 1475*w*, 1425*m*, 1369*w*, 1337*s*, 1312*m*, 1280*m*, 1245*m*, 1187*m*, 1144*m*, 1094*m*, 1060*w*, 1032*w*, 923*w*, 832*w*, 745*w*.

### Beispiel 2: Bestimmung der Aktivität von PAF-AH unter Verwendung der erfindungsgemäßen Verbindung 1-O-{10-[4-(Pyren-1-yl)-butoxy]-decyl}-2-(6-(2,4-dinitrophenyl)-aminohexoyl)-rac-glycero-3-phosphocholin

Für die Untersuchungen wurden 10 mM DMSO-Stammlösung der erfindungsgemäßen Verbindung von Beispiel 1 hergestellt und dann jeweils für die entsprechenden Versuche mit dem verwendeten Puffer (0.1 M TRIS HCI, 0.1 % Tween20, pH 7.4) in den gewünschten Konzentrationen verdünnt.

### Messungen in einer Quarzküvette

Verwendet werden Quarzküvetten (1 cm x 1 cm) der Fa. Helma (Müllheim / Baden, Deutschland), die Füllmenge beträg jeweis 1 ml.
Einstellungen in der Software des Cary Eclipse der Fa. Varian (Darmstadt, Deutschland):
- Im Kinetik-Modul: Ex. Wavelength (nm) = 344.00; Em. Wavelength (nm) = 377.00; Ex. Slit (nm) = 5; Em. Slit (nm) = 5; Averaging Time (s) = 0.1000; Dwell time (s) = 0.1000; Cycle time (min) = 0.2500; Excitation filter = Auto; Emission filter = Open; Multicell holder = Multicell; PMT voltage (V) = Medium; Temperature control.
Die Untersuchungen wurden mit einem Peltier-Vierfach-Küvettenhalter bei 37°C durchgeführt.

### Messungen in einer Microtiterplatte

Verwendet wurden weiße Titerplatten mit 96 Vertiefungen der Fa. Perkin Elmer (Überlingen, Deutschland) und schwarze FIA-Platten mit 96 Vertiefungen der Fa. Greiner (Frikenhausen, Deutschland).
Einstellungen in der Software des Cary Eclipse der Fa. Varian (Darmstadt, Deutschland):
- Im Kinetik-Modul: Ex. Wavelength (nm) = 344.00; Em. Wavelength (nm) = 377.00; Ex. Slit (nm) = 5; Em. Slit (nm) = 5; Averaging Time (s) = 0.1000; Cycle time (min) = 0.0000; Excitation filter = Auto; Emission filter = Open; PMT voltage (V) = Medium; Wellplate = ON; Read position = Well centre.

### Präparation von PAF-AH

### Präparation und Reinheitsüberprüfung von Plasma und von humanem LDL

Verwendeter Puffer: PBS-Puffer (150 mM NaCI, 40 mM Phosphat, 1mM EDTA, pH = 7.4). Der Puffer wurde wurde 15 min lang mit Argon gesättigt.

### Präparation des Plasma

100 ml frisch abgenommenes heparinisiertes Blut wurde in 2 x 50 ml Tubes bei Raumtemperatur 10 min mit der Hermle-Zentrifuge bei 3000 U/min zentrifugiert. Vom Überstand wurden jeweils 20 ml abgenommen, gepoolt, mit 400 µl einer 0.1 M EDTA-Lösung pH = 7.45 versetzt und mit Argon überschichtet.

### Präparation des LDL

32 ml der Plasmaprobe wurden mit 12.2112 g KBr p.a. versetzt und mit Argon überschichtet. Durch langsames Schwenken wurde das KBr gelöst. In 6 x 4 ml Zentrifugenröhrchen der Fa. Beckman (München, Deutschland) wurden 2 ml der PBS-Lösung vorgelegt und mit 1 ml der mit KBr versetzten Plasmaprobe unterschichtet. Diese wurden nun bei 4°C und 37000 rpm im Vakuum 2 Stunden zentrifugiert. Nach dieser Zeit wurden die Zentrifugenröhrchen vorsichtig aus dem Rotor entfernt. Das gewünschte LDL ist als gelbe Bande ca. 1/3 vom Boden des Zentrifugenröhrchens entfernt zu erkennen. Zunächst wurden die obere weiße Schicht abgenommen und verworfen. Die gelbe Schicht ist ca. 1 mm dick. Diese wurde mit einer stumpfen, abgeknickten Kannüle abgenommen. Das LDL aller 6 Zentrifugenröhrchen wurde gepoolt und mit Argon überschichtet Zur Entfernung des KBr wurden 300 µl der so gewonnenen Lösung durch eine Sephadex-G25 Minisäule mit PBS-Puffer chromatographiert.

### Proteinbestimmung

Lösung A: 1 % Na₂BCA, 2 % Na₂CO₃ H₂O, 0.16 % Na₂Tartrat, 0.4 % NaOH, 0.95 % NaHCO₃, pH = 11.25
Lösung B: 4 % CuSO₄ 5 H₂O
Als Standard wurde BSA verwendet; alles Bestimmungen wurden als Doppelbestimmung durchgeführt. Das Gesamtvolumen eines Ansatzes betrug 300 µl / Well, wobei in der Regel 2.5 µl der zu bestimmenden Probe (eventuell nach Verdünnung) eingesetzt wurden. Diese wurden mit Wasser auf 75 µl aufgefüllt und mit 225 µl einer 50:1 Mischung von Lösung A und Lösung B versehen. Die Inkubationszeit betrug 2 Stunden bei Raumtemperatur. Die Proben wurden anschließend photometrisch bei 562 nm vermessen.

### Überprüfung der Reinheit des isolierten LDL

Die Überprüfung wurde mittels submaritimer Mini-Gelelektrophorese durchgeführt.
- Gelzusammensetzung: 20 ml Barbitalpuffer (50 mM, pH = 8.6), 0.3 % Separide™ (60 mg)
- Vorbereitung des isolierten LDL: 10 µl (0.5 - 1 µg) des gesäulten LDL, 0.5 µl 9-Diethylamino-5H[α]phenoxazin-5-on (Nilrot) (0.4 mg/ml), 10 µl Auftragepuffer (TBE-Puffer, 30 % Glycerin, 1 % SDS, Spatelspitze Bromphenolblau)
- TBE-Puffer (89 mM TRIS HCI, 89 mM B(OH)₃, 2 mM EDTA, pH = 8.5)
- Dauer der Elektrophorese: 1 h (bei konstantem U von 56 V) bei Raumtemperatur
- Flixierlösung: 25 % ⁱPrOH, 10 % Essigsäure, 65 % Wasser
- Färbelösung: 50 % MeOH, 10 % Essigsäure, 40 % Wasser, 0.05 % Coomassie brilliant blue R 250
- Entfärbelösung: 5 % MeOH, 7 % Essigsäure, 88 % Wasser

### Isolierung der PAF-AH aus LDL

Puffer A: 25 mM TRIS HCI, 0.1 % Tween20, 2 mM EDTA, pH 7.6
Puffer B: 25 mM TRIS HCI, 0.1 % Tween20, 2 mM EDTA, 50 - 200 mM NaCl, pH 7.6 (linearer Gradient)
Alle Arbeiten wurden bei 4°C durchgeführt.

Es wurden 1 ml (2 mg/ml) LDL auf eine vorequilibrierte (Puffer A) DEAE Sepharose® FF-Chromatographiesäule der Fa. Amersham Pharmacia biotech (Freiburg, Deutschland) pipetiert und mit dem 6-fachen Säulenfüllvolumen bis zum proteinfreien Eluat gewaschen. Anschließend wurde das Protein mit Puffer B eluiert. Die höchste PAF-AH Aktivität wurde bei den Fraktionen von 100 -150 mM NaCI erhalten. Diese wurden gepoolt und durch Ultrafiltration (Amicon YM30) eingeengt. Die aufkonzentrierten Fraktionen wurden sodann auf eine vorequilibrierte (Puffer A) blue Sepharose® 6FF-Chromatographiesäule der Fa. Amersham Pharmacia biotech (Freiburg, Deutschland) pipetiert und mit dem 6-fachen Säulenfüllvolumen bis zum proteinfreien Eluat gewaschen. Anschließend wurde das Protein mit Puffer A + 0.5 M NaCI eluiert. Die Fraktionen mit der höchsten PAF-AH Aktivität wurden gepoolt und durch Ultrafiltration (Amicon YM30) eingeengt.

Der Gehalt an PAF-AH wurde durch Proteinbestimmung durchgeführt. Die Reinheit wurden in Anlehnung an die SDS Elektrophorese des LDL untersucht. Die erhaltene PAF-AH war für die Aktivitätsuntersuchungen ausreichend rein.

In Fig. 1 ist der zeitliche Verlauf der Fluoreszenzzunahme durch die Einwirkung von PAF-AH auf die erfindungsgemäße Verbindung 1-O-{10-[4-(Pyren-1-yl)-butoxy]-decyl}-2-(6-(2,4-dinitrophenyl)-aminohexoyl)-*rac*-glycero-3-phosphocholin. Die Messung wurde unter vorstehend beschriebenen Bedingungen durchgeführt. Wie Fig. 1 entnommen werden kann, erfolgt eine Zunahme der Fluoreszenz in Anwesenheit von PAF-AH. Ohne PAF-AH (Kontrolle) war keine Zunahme der Fluoreszenzintensität zu beobachten.

In Fig. 2 ist der Zusammenhang zwischen unterschiedlicher Menge von PAF-AH bzw. unterschiedlichen Verdünnungen von humanem Probenmaterial und der Zunahme der Fluoreszenzintensität dargestellt (Versuchsbedingungen vgl. vorstehend). Dabei wurde festgestellt, daß bei höheren PAF-AH-Konzentrationen eine verstärkte Zunahme des Fluoreszenzintensität zu beobachten ist.

## Patentansprüche

1. Verbindung der Formel (1) 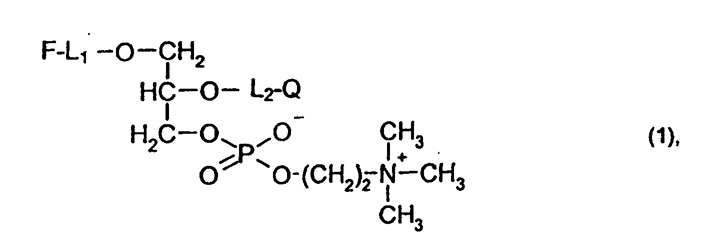 in der
L₁ von einem Ether (R¹-OR²)ₘ, wobei R¹ und R² unabhängig voneinander gewählt und von einem Kohlenwasserstoff mit 1 bis 12 Kohlenstoffatomen abgeleitet sind, wobei m eine ganze Zahl von 1 bis 4 ist, oder von einem Kohlenwasserstoff R mit 1 bis 20 Kohlenstoffatomen abgeleitet ist;
F für ein unsubstituiertes oder substituiertes Pyren als Fluorophor;
Q für einen Quencher und
L₂ für C(O)-L₁ oder C(O)-L₁-NH, wobei L₁ wie vorstehend definiert ist, stehen.

2. Verbindung nach einem der vorhergehenden Ansprüche, wobei der Quencher der 2,4-Dinitrophenyl-Rest ist.

3. Verbindung nach einem der vorhergehenden Ansprüche, wobei R¹ und R² unabhängig voneinander für (CH₂)ₙ stehen und n eine ganze Zahl von 2 bis 12 ist.

4. Verbindung nach einem der vorhergehenden Ansprüche, wobei L₁ für (CH₂)₄-O-(CH₂)₁₀ steht.

5. Verbindung nach einem der Ansprüche 1 oder 2, wobei R für (CH₂)ₒ steht und o eine ganze Zahl von 1 bis 20 ist

6. Verbindung nach einem der vorhergehenden Ansprüche, wobei L₂ für C(O)-(CH₂)ₚ oder C(O)-(CH₂)ₚ-NH steht und p eine ganze Zahl von 1 bis 20 ist.

7. Verbindung nach Anspruch 6, wobei L₂ für C(O)-(CH₂)₅-NH steht.

8. Verbindung nach einem der vorhergehenden Ansprüche, wobei sie die Struktur der Formel (2) aufweist

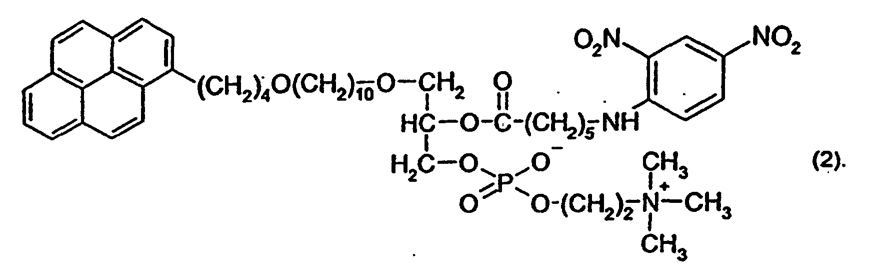

9. Verwendung der Verbindung nach einem der vorhergehenden Ansprüche zur Bestimmung der Aktivität von Phospholipase A₂.

10. Verwendung nach Anspruch 9, wobei die Phospholipase A₂ die Platelet-Activating Factor Acetylhydrolase ist.

## Claims

1. Compound of formula (1) 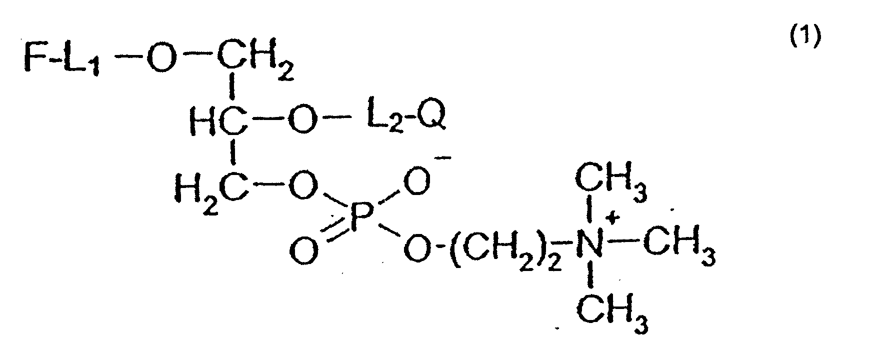 wherein
L₁ is derived from an ether (R¹-OR²)ₘ, wherein R¹ and R² are independent from one another and are derived from hydrocarbon containing 1 to 12 carbon atoms, with m being an integer from 1 to 4, or from a hydrocarbon R containing 1 to 20 carbon atoms;
F is an unsubstituted or substituted pyrene as fluorophore;
Q is a quencher, and
L₂ is C(O)- L₁ or C(O)- L₁-NH, wherein, L₁ is defined as above.

2. Compound according to any one of the preceding claims, wherein the quencher is the 2,4-dinitrophenyl residue.

3. Compound according to any one of the preceding claims, wherein R¹ and R² are each independently from one another (CH₂)ₙ and n is an integer from 2 to 12.

4. Compound according to any one of the preceding claims, wherein L₁ is (CH₂)₄-O-(CH₂)₁₀.

5. Compound according to one of Claims 1 or 2, wherein R is (CH₂)ₒ and o is an integer from 1 to 20.

6. Compound according to any one of the preceding claims, wherein L₂ is C(O)-(CH₂)ₚ or C(O)-(CH₂)ₚ-NH and p is an integer from 1 to 20.

7. Compound according to Claim 6, wherein L₂ is C(O)-(CH₂)₅-NH.

8. Compound according to any one of the preceding claims having the structure of formula 2

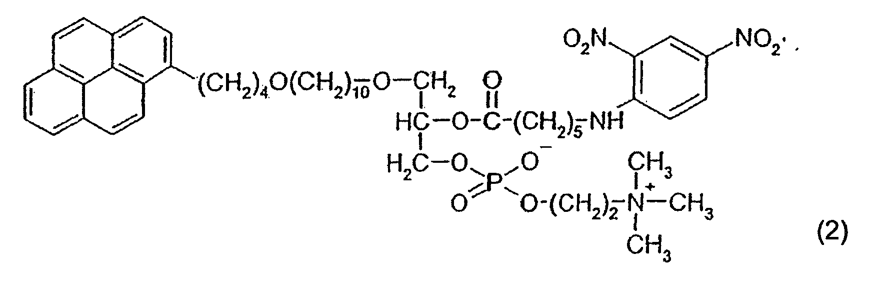

9. Use of the compound according to any one of the preceding claims, for the determination of the activity of Phospholipase A₂.

10. Use of the compound according to claim 9, wherein phospholipase A₂ is the platelet-activating factor acetylhydrolase.

## Revendications

1. Composé de formule (1) 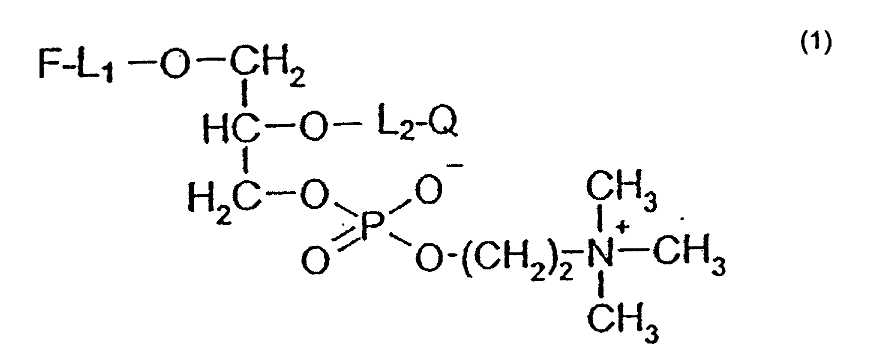 dans laquelle :
L₁ est dérivé d'un éther (R¹-OR²)ₘ où R¹ et R² sont choisis indépendamment et sont dérivés d'une hydrocarbure comprenant 1 à 12 atomes de carbone, où m est un nombre entier compris entre 1 et 4, ou d'une hydrocarbure R comprenant 1 à 20 atomes de carbone ;
F représente un pyrène substitué ou non substitué tenant lieu de fluorophore
Q est un désactiveur et
L₂ représente C(O)- L₁ ou C(O)- L₁-NH, avec, L₁ étant tel que défini ci-dessus.

2. Composé selon l'une quelconque des revendications ci-dessus, **caractérisé en ce que** le désactiveur est le résidu 2,4-dinitrophenyl.

3. Composé selon l'une quelconque des revendications ci-dessus, **caractérisé en ce que** R¹ et R² sont chacun indépendant vis-à-vis de (CH₂)ₙ, et n est un nombre entier compris entre 2 et 12.

4. Composé selon l'une quelconque des revendications ci-dessus, **caractérisé en ce que** L₁ représente (CH₂)₄-O-(CH₂)₁₀.

5. Composé selon l'une des revendications 1 ou 2, **caractérisé en ce que** R représente (CH₂)ₒ, où o est un nombre entier compris entre 1 et 20.

6. Composé selon l'une quelconque des revendications ci-dessus, **caractérisé en ce que** L₂ représente C(O)-(CH₂)ₚ ou C(O)-(CH₂)ₚ-NH et p est un nombre entier compris entre 1 et 20.

7. Composé selon la revendication 6, **caractérisé en ce que** L₂ représente C(O)-(CH₂)₅-NH.

8. Composé selon l'une quelconque des revendications ci-dessus, **caractérisé en ce que** sa structure dérive de la formule 2.

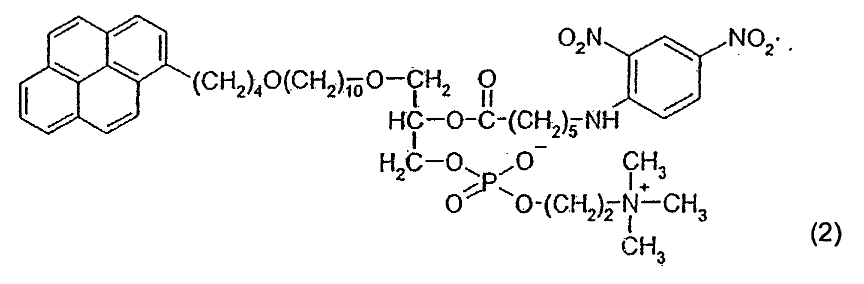

9. Utilisation du composé selon l'une quelconque des revendications ci-dessus, dans la détermination de l'activité de la phospholipase A₂.

10. Utilisation selon la revendication 9, **caractérisé en ce que** la phospholipase A₂ représente le facteur d'activation de plaquettes acetylhydrolase.
